# EUROPEAN PATENT APPLICATION

(11) **EP 4 693 312 A1**
(43) Date of publication of application: **11.02.2026**
(21) Application number: 25193321.4
(22) Date of filing: 01.08.2025
(51) Int. Cl.: G16C 60/00, G16C 20/40, G16C 20/90

(54) **METHOD AND SYSTEM FOR SCREENING MATERIAL FOR LITHIUM SECONDARY BATTERY**

(30) Priority: 08.08.2024 KR 20240106026
(71) Applicant: SAMSUNG SDI CO., LTD., Yongin-si, Gyeonggi-do 17084 (KR)
(72) Inventor: SUH, Seung Bum, 17084 Yongin-Si, Gyeonggi-do (KR); KIM, Dongyoung, 17084 Yongin-Si, Gyeonggi-do (KR); SINGH, Jiten, 17084 Yongin-Si, Gyeonggi-do (KR); KIM, Soojin, 17084 Yongin-Si, Gyeonggi-do (KR); KIM, Hyeonsu, 17084 Yongin-Si, Gyeonggi-do (KR); LEE, Kyung Min, 17084 Yongin-Si, Gyeonggi-do (KR); KIM, Seulwoo, 17084 Yongin-Si, Gyeonggi-do (KR); LEE, Sunmin, 17084 Yongin-Si, Gyeonggi-do (KR)
(74) Representative: Michalski Hüttermann & Partner Patentanwälte mbB

(57) **Abstract**

A method and system for screening materials for lithium secondary batteries. The method includes receiving information about organic substances, generating a database by storing the information about the organic substances based on a plurality of parameters, and applying a filter to the database to generate output information about a target substance configured to be used in the lithium secondary battery, the target substance selected from the organic substances, wherein the target substance is configured to form at least a part of a low-resistance solid electrolyte interphase (SEI) layer on an anode of the lithium secondary battery.

## Description

### BACKGROUND

### 1. Field

Embodiments of the present disclosure relate to a method and system for screening materials for lithium secondary batteries that outputs information on a target substance that forms at least a part of a low-resistance SEI (solid electrolyte interphase) layer on the anode of a lithium secondary battery.

### 2. Description of the Related Art

While primary batteries are not designed to be (re)charged, secondary (also known as rechargeable) batteries are batteries that are designed to be discharged and recharged. Among secondary batteries, low-capacity secondary batteries are widely used in portable, small electronic devices, such as smart phones, feature phones, notebook computers, digital cameras, and camcorders, while high-capacity secondary batteries are widely used as power sources for driving motors in hybrid vehicles and electric vehicles, as well as for storing power (e.g., home and/or utility scale power storage). A secondary battery generally includes an electrode assembly including a positive electrode and a negative electrode, a case accommodating both electrodes, and electrode terminals connected to the electrode assembly.

Secondary batteries typically house an electrolyte for lithium ions to migrate within. Various materials can be used as an electrolyte, but due to reasons such as environmental, process safety, yield improvement, or other reasons, there is a necessity to use electrolyte materials different from those used previously. For example, as secondary batteries continue to be used, they can deteriorate, resulting in a reduction in service life. Accordingly, electrolyte materials need to be replaced in order to inhibit the deterioration.

Conducting experiments to find candidate materials configured to be used in the electrolyte is experimentally time consuming, and requires a great degree of effort and resources. In fact, finding candidate electrolyte materials meeting their requisite conditions is a great challenge for secondary battery manufacturers.

This Background section is for the general understanding of the background of the present disclosure, and therefore, it may contain information that does not constitute related (or prior) art.

### SUMMARY

It is an object of the present disclosure to provide a solution to the above-mentioned technical challenges.

Embodiments of the present disclosure provide a method and system for screening materials for lithium secondary batteries.

These and other aspects and features of the present disclosure will be described in or will be apparent from the following description of embodiments of the present disclosure.

According to one or more embodiments of the present disclosure , a method of screening materials for a lithium secondary battery includes receiving information on organic substances, generating a database by storing the information on the organic substances based on a plurality of parameters, and outputting information on a target substance to be used in a lithium secondary battery out of the organic substances by applying at least one filters to the database, wherein the target substance forms at least a part of a low-resistance solid electrolyte interphase (SEI) layer on an anode of the lithium secondary battery.

Embodiments of the present disclosure provide a method of screening materials configured to be used in a lithium secondary battery, including: receiving information about one or more organic substances; generating a database by storing the information about the one or more organic substances based on a plurality of parameters; and applying at least one filter to the database to generate output information about a target substance configured to be used in the lithium secondary battery, the target substance selected from the one or more organic substances, wherein the target substance is configured to form at least a part of a low-resistance solid electrolyte interphase (SEI) layer on an anode of the lithium secondary battery.

The method is preferably a computer-implemented method and/or carried out by a computer.

According to one or more embodiments of the present disclosure, the plurality of parameters may include parameters associated with at least one of an ion type, a molecular weight, an electron affinity, an ionization energy, a lithium-ion (Li⁺) interaction energy, a LUMO (lowest unoccupied molecular orbital) energy, a LUMO energy of a lithium-ion complex containing the organic substance, or an electron affinity of the lithium-ion complex containing the organic substance.

In some embodiments, the plurality of parameters includes an ion type, a molecular weight, an electron affinity, an ionization energy, a lithium-ion (Li⁺) interaction energy, a LUMO (lowest unoccupied molecular orbital) energy, a LUMO energy of a lithium-ion complex containing the one or more organic substances, or an electron affinity of the lithium-ion complex containing the one or more organic substances.

According to one or more embodiments of the present disclosure, the at least one filters may include a first filter, and the first filter may filter out materials for which an ionization energy of a material included in the database is greater than an ionization energy threshold, out of materials included in the database.

In some embodiments, the at least one filter includes a first filter, and wherein the first filter filters out a candidate material having an ionization energy greater than a predetermined ionization energy threshold.

According to one or more embodiments of the present disclosure, the ionization energy threshold may be associated with an ionization energy of a representative organic solvent, which is an organic solvent of the lithium secondary battery.

In some embodiments, the predetermined ionization energy threshold is related to an ionization energy of an organic solvent previously determined to be used in the lithium secondary battery.

According to one or more embodiments of the present disclosure, the at least one filters may include a second filter, and the second filter may filter out materials for which a lithium-ion interaction energy of a material included in the database is greater than a first lithium-ion interaction energy threshold, out of materials included in the database.

In some embodiments, the at least one filter includes a second filter, and wherein the second filter filters out a candidate material having a lithium-ion interaction energy greater than a first predetermined lithium-ion interaction energy threshold.

According to one or more embodiments of the present disclosure, the first lithium-ion interaction energy threshold may be associated with a lithium-ion interaction energy of a representative organic solvent, which is an organic solvent of the lithium secondary battery.

In some embodiments, the first predetermined lithium-ion interaction energy threshold is related to a lithium-ion interaction energy of an organic solvent previously determined to be used in the lithium secondary battery.

According to one or more embodiments of the present disclosure, the at least one filters may include a third filter, and the third filter may filter out materials for which a lithium-ion interaction energy of a material included in the database is less than a second lithium-ion interaction energy threshold, out of materials included in the database.

In some embodiments, the at least one filter includes a third filter, and wherein the third filter filters out a candidate material having a lithium-ion interaction energy less than a second predetermined lithium-ion interaction energy threshold.

According to one or more embodiments of the present disclosure, the second lithium-ion interaction energy threshold may be associated with an interaction energy between a representative additive, which is an electrolyte additive of the lithium secondary battery, and lithium ions.

In some embodiments, the second predetermined lithium-ion interaction energy threshold is related to an interaction energy between an electrolyte additive previously determined to be used in the lithium secondary battery, and lithium ions.

According to one or more embodiments of the present disclosure, the at least one filters may include a fourth filter, and the fourth filter may filter out materials for which a LUMO energy of a lithium-ion complex containing a material included in the database is less than a LUMO energy threshold, out of materials included in the database.

In some embodiments, the at least one filter includes a fourth filter, and wherein the fourth filter filters out a candidate material having a LUMO energy of a lithium-ion complex less than a predetermined LUMO energy threshold.

According to one or more embodiments of the present disclosure, the LUMO energy threshold may be associated with a LUMO energy of a representative lithium-ion complex containing a representative organic solvent, which is an organic solvent of the lithium secondary battery.

In some embodiments, the predetermined LUMO energy threshold is related to a LUMO energy of a lithium-ion complex comprising an organic solvent previously determined to be used in the lithium secondary battery.

According to one or more embodiments of the present disclosure, the at least one filters may include a fifth filter, the fifth filter may filter out materials for which an electron affinity of a lithium-ion complex containing a material included in the database is greater than an electron affinity threshold, out of materials included in the database, and the electron affinity threshold may be associated with an electron affinity threshold of a representative lithium-ion complex containing a representative organic solvent, which is an organic solvent of the lithium secondary battery.

In some embodiments, the at least one filter includes a fifth filter, wherein the fifth filter filters out a candidate material having an electron affinity of a lithium-ion complex greater than a predetermined electron affinity threshold, and wherein the electron affinity threshold is related to an electron affinity threshold of a lithium-ion complex comprising an organic solvent previously determined to be used in the lithium secondary battery.

According to one or more embodiments of the present disclosure, the representative organic solvent may be one of ethylene carbonate (EC), fluoroethylene carbonate (FEC), ethyl methyl carbonate (EMC), dimethyl carbonate (DMC), ethyl propionate (EP), or ethyl acetate (EA).

In some embodiments, the organic solvent is ethylene carbonate (EC), fluoroethylene carbonate (FEC), ethyl methyl carbonate (EMC), dimethyl carbonate (DMC), ethyl propionate (EP), or ethyl acetate (EA).

According to one or more embodiments of the present disclosure, the method of screening materials for a lithium secondary battery may further include receiving an input that selects at least one of the plurality of parameters and receiving information on a numerical range for the selected parameter, wherein the at least one filters may include a sixth filter, and the sixth filter may filter out materials for which information associated with at least one of the selected plurality of parameters of a material included in the database falls within the numerical range, out of materials included in the database.

In some embodiments, the method further includes: receiving an input for selecting at least one of the plurality of parameters; and receiving information about a numerical range for the at least one of the plurality of parameters, wherein the at least one filter comprises a sixth filter, and wherein the sixth filter filters out a candidate material in which the numerical range comprises information related to the at least one of the plurality of parameters.

According to one or more embodiments of the present disclosure, the outputting the information on the target substance may include receiving an input that has selected at least one of the plurality of parameters on a user interface, and outputting information on at least one of the selected plurality of parameters out of the information on the target substance onto the user interface.

In some embodiments, the generating of the output information includes: receiving an input of selecting at least one of the plurality of parameters on a user interface; and generating output information about the at least one of the plurality of parameters selected from the output information about the target substance onto the user interface.

There may be provided a computer program stored on a computer-readable recording medium for executing the method according to one or more embodiments of the present disclosure on a computer. Preferably a computer program stored on a computer-readable recording medium is provided, the computer program comprising instructions which, when the program is executed by a computer, cause the computer to execute the above-described method.

Embodiments of the present disclosure provide a computer program stored on a computer-readable recording medium for executing any of the preceding methods on a computer.

According to one or more embodiments of the present disclosure, an information processing system includes a communication module, a memory, and at least one processor connected to the memory and configured to execute at least one computer-readable program included in the memory, wherein the at least one program includes instructions for receiving information on organic substances, generating a database by storing the information on the organic substances based on a plurality of parameters, and outputting information on at least one target substances to be used in a lithium secondary battery out of the organic substances by applying at least one filters to the database, and wherein the target substance forms at least a part of a low-resistance SEI layer on an anode of the lithium secondary battery.

Preferably, the at least one program is configured as described above and/or causes the information processing system to execute the above-described method of screening materials configured to be used in a lithium secondary battery.

Embodiments of the present disclosure provide an information processing system including: a communication module; a memory; and at least one processor connected to the memory and configured to execute at least one computer-readable program comprised in the memory, wherein the at least one program includes instructions for: receiving information about one or more organic substances; generating a database by storing the information about the one or more organic substances based on a plurality of parameters; and applying at least one filter to the database to generate output information about a target substance configured to be used in the lithium secondary battery, the target substance selected from the one or more organic substances, wherein the target substance is configured to form at least a part of a low-resistance solid electrolyte interphase (SEI) layer on an anode of the lithium secondary battery.

According to one or more embodiments of the present disclosure, the at least one filters may include a first filter, and the first filter may filter out materials for which an ionization energy of a material included in the database is greater than an ionization energy threshold, out of materials included in the database.

In some embodiments, the at least one filter includes a first filter, and wherein the first filter filters out a candidate material having an ionization energy greater than a predetermined ionization energy threshold.

According to one or more embodiments of the present disclosure, the at least one filters may include a second filter, and the second filter may filter out materials for which a lithium-ion interaction energy of a material included in the database is greater than a first lithium-ion interaction energy threshold, out of materials included in the database.

In some embodiments, the at least one filter includes a second filter, and wherein the second filter filters out a candidate material having a lithium-ion interaction energy greater than a first predetermined lithium-ion interaction energy threshold.

According to one or more embodiments of the present disclosure, the at least one filters may include a third filter, and the third filter may filter out materials for which a lithium-ion interaction energy of a material included in the database is less than a second lithium-ion interaction energy threshold, out of materials included in the database.

In some embodiments, the at least one filter includes a third filter, and wherein the third filter filters out a candidate material having a lithium-ion interaction energy less than a second predetermined lithium-ion interaction energy threshold.

According to one or more embodiments of the present disclosure, the at least one filters may include a fourth filter, and the fourth filter may filter out materials for which a LUMO energy of a lithium-ion complex containing a material included in the database is less than a LUMO energy threshold, out of materials included in the database.

In some embodiments, the at least one filter includes a fourth filter, and wherein the fourth filter filters out a candidate material having a LUMO energy of a lithium-ion complex less than a predetermined LUMO energy threshold.

According to various embodiments of the present disclosure, when a name that can specify an organic substance is input into the system for screening materials for a lithium secondary battery, the system for screening materials for a lithium secondary battery can generate information on the organic substance and automatically build a database based on the information on the organic substance.

According to various embodiments of the present disclosure, organic substances to be used in secondary batteries can be readily searched for using the database without any other experiments. Moreover, it may take a lot of time and effort to find a material that inhibits the deterioration of secondary batteries, but because there is no need to perform repetitive experiments to find a material (e.g., organic substance) that inhibits the deterioration of a secondary battery, time, effort, resources, costs, etc., can be saved. For example, in a lithium secondary battery, an SEI layer can be formed on the anode as the secondary battery is charged and discharged. The secondary battery can be deteriorated due to the electric resistance of the SEI layer. In this case, by obtaining information on organic substances that form an SEI layer with relatively lower resistance than the SEI layer formed previously, organic substances that slow down or inhibit the deterioration of lithium secondary batteries can be readily searched for.

According to various embodiments of the present disclosure, organic substances that form at least a part of the low-resistance SEI layer on the anode of the lithium secondary battery can be screened using at least some of the plurality of filters.

According to various embodiments of the present disclosure, filters can be readily generated via the user interface, and target substances that can inhibit the deterioration of the lithium secondary battery can be readily searched for using the generated filters.

According to various embodiments of the present disclosure, by allowing the user to select at least one of the plurality of parameters and to input a numerical range for the selected parameter to generate a filter, organic substances suitable for various conditions of the lithium secondary battery can be searched for.

According to various embodiments of the present disclosure, the information on the target substances can be displayed with high readability via the user interface. The user can compare the substances included in the target substances with each other via the user interface, and more suitable substances can be selected.

### BRIEF DESCRIPTION OF DRAWINGS

The drawings illustrate embodiments of the present disclosure, and further describe aspects and features of the present disclosure along with the detailed description of the present disclosure. Thus, the present disclosure should not be construed as being limited to the drawings:
FIG. 1 is a schematic diagram showing a system for screening materials for a lithium secondary battery according to embodiments of the present disclosure;
FIG. 2 is a schematic diagram showing a configuration in which an information processing system is communicably connected to a plurality of user terminals for screening materials for a lithium secondary battery according to embodiments of the present disclosure;
FIG. 3 is a block diagram showing an internal configuration of a user terminal and an information processing system according to embodiments of the present disclosure;
FIG. 4 is a cross-sectional view showing a lithium secondary battery according to embodiments of the present disclosure;
FIG. 5 shows a cathode, an anode, and an electrolyte according to embodiments of the present disclosure;
FIG. 6 shows a cathode, an anode, and an electrolyte according to embodiments of the present disclosure;
FIG. 7 shows example information about organic substances according to embodiments of the present disclosure;
FIG. 8 is a flowchart showing an example method for screening materials for a lithium secondary battery according to embodiments of the present disclosure;
FIG. 9 shows generating output information about a target substance using a plurality of filters according to embodiments of the present disclosure;
FIG. 10 shows an example user interface onto which information on target substances is output according to embodiments of the present disclosure; and
FIG. 11 shows an example filter input interface according to embodiments of the present disclosure.

### DETAILED DESCRIPTION

Hereinafter, embodiments of the present disclosure will be described, in detail, with reference to the accompanying drawings. The terms or words used in this specification and claims should not be construed as being limited to the usual or dictionary meaning and should be interpreted as meaning and concept consistent with the technical idea of the present disclosure based on the principle that the inventor can be his/her own lexicographer to appropriately define the concept of the term to explain his/her invention in the best way.

The embodiments described in this specification and the configurations shown in the drawings are only some of the embodiments of the present disclosure and do not represent all of the technical ideas, aspects, and features of the present disclosure. Accordingly, it should be understood that there may be various equivalents and modifications that can replace or modify the embodiments described herein at the time of filing this application.

It will be understood that when an element or layer is referred to as being "on," "connected to," or "coupled to" another element or layer, it may be directly on, connected, or coupled to the other element or layer or one or more intervening elements or layers may also be present. When an element or layer is referred to as being "directly on," "directly connected to," or "directly coupled to" another element or layer, there are no intervening elements or layers present. For example, when a first element is described as being "coupled" or "connected" to a second element, the first element may be directly coupled or connected to the second element or the first element may be indirectly coupled or connected to the second element via one or more intervening elements.

To facilitate understanding of the disclosure, the attached drawings are not drawn to actual scale and the dimensions of some components may be exaggerated. Furthermore, the same reference numbers may be assigned to the same components in different embodiments. As used herein, the term "and/or" includes any and all combinations of one or more of the associated listed parameters. Further, the use of "may" when describing embodiments of the present disclosure relates to "one or more embodiments of the present disclosure." Expressions, such as "at least one of" and "any one of," when preceding a list of elements, modify the entire list of elements and do not modify the individual elements of the list. When phrases such as "at least one of A, B and C, "at least one of A, B or C," "at least one selected from a group of A, B and C," or "at least one selected from among A, B and C" are used to designate a list of elements A, B and C, the phrase may refer to any and all suitable combinations or a subset of A, B and C, such as A, B, C, A and B, A and C, B and C, or A and B and C. As used herein, the terms "use," "using," and "used" may be considered synonymous with the terms "utilize," "utilizing," and "utilized," respectively. As used herein, the terms "substantially," "about," and similar terms are used as terms of approximation and not as terms of degree, and are intended to account for the inherent variations in measured or calculated values that would be recognized by those of ordinary skill in the art.

It will be understood that, although the terms first, second, third, etc. may be used herein to describe various elements, components, regions, layers, and/or sections, these elements, components, regions, layers, and/or sections should not be limited by these terms. These terms are used to distinguish one element, component, region, layer, or section from another element, component, region, layer, or section. Thus, a first element, component, region, layer, or section discussed below could be termed a second element, component, region, layer, or section without departing from the teachings of example embodiments.

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" or "over" the other elements or features. Thus, the term "below" may encompass both an orientation of above and below. The device may be otherwise oriented (rotated 90 degrees or at other orientations), and the spatially relative descriptors used herein should be interpreted accordingly.

The terminology used herein is for the purpose of describing embodiments of the present disclosure and is not intended to be limiting of the present disclosure. As used herein, the singular forms "a" and "an" are intended to include the plural forms as well, unless the context clearly indicates otherwise. It will be further understood that the terms "includes," "including," "comprises," and/or "comprising," when used in this specification, specify the presence of stated features, integers, steps, operations, elements, and/or components but do not preclude the presence or addition of one or more other features, integers, steps, operations, elements, components, and/or groups thereof.

Also, any numerical range disclosed and/or recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein, and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein.

References to two compared elements, features, etc. as being "the same" may mean that they are "substantially the same". Thus, the phrase "substantially the same" may include a case having a deviation that is considered low in the art, for example, a deviation of 5% or less. In addition, when a certain parameter is referred to as being uniform in a given region, it may mean that it is uniform in terms of an average.

Throughout the specification, unless otherwise stated, each element may be singular or plural.

Arranging an arbitrary element "above (or below)" or "on (under)" another element may mean that the arbitrary element may be disposed in contact with the upper (or lower) surface of the element, and another element may also be interposed between the element and the arbitrary element disposed on (or under) the element.

In addition, it will be understood that when a component is referred to as being "linked," "coupled," or "connected" to another component, the elements may be directly "coupled," "linked" or "connected" to each other, or another component may be "interposed" between the components".

Throughout the specification, when "A and/or B" is stated, it means A, B or A and B, unless otherwise stated. That is, "and/or" includes any or all combinations of a plurality of parameters enumerated. When "C to D" is stated, it means C or more and D or less, unless otherwise specified.

Cases where the information on a material (e.g., the ionization energy of a material included in a database) is greater than or less than a threshold (e.g., an ionization energy threshold) will be described separately, but the present disclosure is not limited thereto. For example, a configuration for filtering out materials included in the database whose ionization energy is greater than an ionization energy threshold may be applied when filtering out materials included in the database whose ionization energy is greater than or equal to the ionization energy threshold. Similarly, a configuration for filtering out materials included in the database whose ionization energy is less than or equal to the ionization energy threshold may be applied when filtering out materials included in the database whose ionization energy is less than the ionization energy threshold.

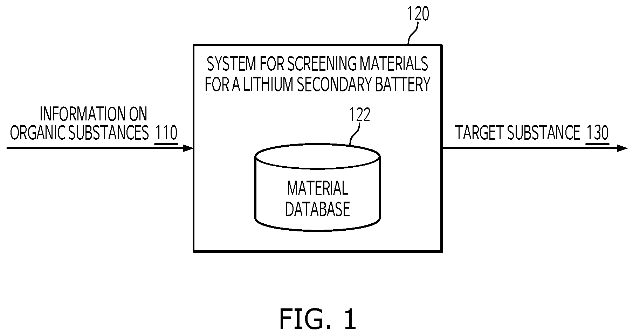

FIG. 1 is a schematic diagram showing a system 120 for screening materials for a lithium secondary battery according to embodiments of the present disclosure. The system 120 for screening materials for a lithium secondary battery may receive information 110 about an organic substance. The organic substance may be contained in an electrolyte for a lithium secondary battery. In some embodiments, the organic substance may be used as an organic solvent in the electrolyte for the lithium secondary battery. In some embodiments, the organic substance may be used as an additive in the electrolyte for the lithium secondary battery.

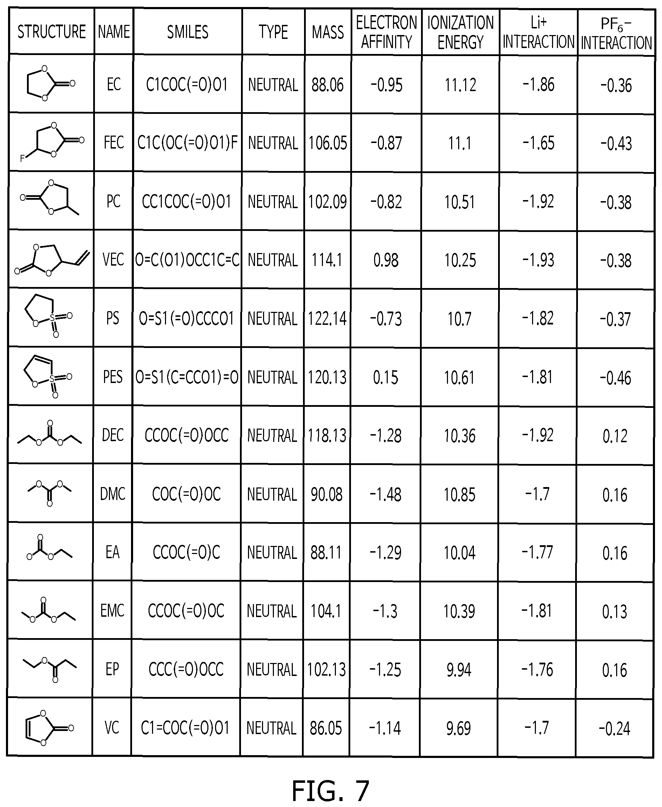

The information 110 about the organic substance may include information about molecular properties and/or chemical properties of the organic substance. In some embodiments, the information 110 about the organic substance may include information related to the ion types (e.g., anionic, cationic, neutral, etc.), molecular weight, dipole moment, electron affinity, and ionization energy of the organic substance, a lithium-ion interaction energy (e.g., an interaction energy for the reaction between the organic substance and lithium ions), an interaction energy between the organic substance and the anions of a lithium salt (e.g., PF₆⁻, BF₄⁻, SbF₆⁻, AsF₆⁻, ClO₄⁻ , AlO₂⁻, AlCl₄⁻, PO₂F₂⁻, Cl⁻, I⁻, etc.), a HOMO (highest occupied molecular orbital) energy, a LUMO (lowest unoccupied molecular orbital) energy, a protonation energy (PE), a dehydrogenation energy (DE), an interaction energy of oxygen radicals, an electron affinity of a lithium-ion complex containing the organic substance, LUMO of the lithium-ion complex containing the organic substance, an interaction energy between the lithium-ion complex containing the organic substance and the oxygen radicals, an interaction energy between the organic substance and a transition metal (e.g., Ni²⁺, Fe²⁺, Co²⁺, Mn², etc.), etc. The interaction energy between the organic substance and the anions of the lithium salt refers to a Gibbs free-energy change for a reaction in which the organic substance bonds with the anions of the lithium salt. The protonation energy refers to a Gibbs free-energy change for a reaction in which the organic substance bonds with protons. The dehydrogenation energy refer to a Gibbs free-energy change for the reaction of dissociating the bond of the hydrogen atoms (or hydrogen radicals or hydrogen molecules) from the organic substance. The interaction energy of the oxygen radicals refers to a Gibbs free-energy change for a reaction in which the organic substance bonds with the oxygen radicals. An example of the organic substance and the information 110 about the organic substance will be described in detail with reference to FIG. 7 and Table 1.

The lithium-ion complex containing the organic substance refers to a complex in which lithium ions (Li⁺) and the organic substance are combined. The interaction energy between the lithium-ion complex containing the organic substance and the oxygen radicals refers to a Gibbs free-energy change according to a reaction in which the lithium-ion complex containing the organic substance and the oxygen radicals are bonded.

In some embodiments, the lithium salt may be contained in the electrolyte for the lithium secondary battery. The lithium salt may be dissolved in an organic solvent contained in the electrolyte, acts as a source of lithium ions in the lithium secondary battery to enable the basic operation of the lithium secondary battery, and serves to facilitate the migration of lithium ions between the cathode and anode. For example, the lithium salt may include one or more selected from LiPF₆, LiBF₄, LiSbF₆, LiAsF₆, LiClO₄, LiAlO₂, LiAlCl₄, LiPO₂F₂, LiCl, Lil, LiN(SO₃C₂F₅)₂, Li(FSO₂)₂N (lithium bis(fluorosulfonyl)imide (LiFSI), LiC₄F₉SO₃, LiN(CₓF₂ₓ₊₁SO₂)(C_{y}F_{2y+1}SO₂) (x and y are integers between 1 and 20), lithium trifluoromethane sulfonate, lithium tetrafluoroethanesulfonate, lithium difluorobis(oxalato)phosphate (LiDFOB), and lithium bis(oxalato)borate (LiBOB).

In one embodiment, the information 110 about the organic substance may include a name capable of specifying an organic substance. In some embodiments, the name that can specify an organic substance may include the name, SMILES (Simplified Molecular Input Line Entry System), CAS number, etc., of a compound. In this case, the system 120 for screening materials for a lithium secondary battery may generate the information 110 on the organic substance, including information on the molecular properties and chemical properties of the organic substance, based on the name capable of specifying the organic substance. In some embodiments, the system 120 for screening materials for a lithium secondary battery may specify an organic substance based on the name capable of specifying the organic substance. The system 120 for screening materials for a lithium secondary battery may generate a molecular geometry of the specified organic substance using DFT (density functional theory) calculation to optimize the molecular geometry. The system 120 for screening materials for a lithium secondary battery may generate information on the molecular properties and chemical properties of the organic substance based on the molecular geometry of the organic substance. Moreover, the system 120 for screening materials for a lithium secondary battery may receive at least some of the information on the molecular properties and chemical properties of the organic substance generated from an external system.

In some embodiments, the information necessary to generate the information on the molecular properties and chemical properties of the organic substance may be determined in advance. In some embodiments, information on the oxygen radicals, information on the lithium ions, information on the anions of the lithium salt, etc., may be determined in advance. Specifically, information on the ion type, molecular weight, electron affinity, ionization energy, molecular geometry, etc., of the oxygen radicals, lithium ions, anions of the lithium salt, etc., may be determined in advance. Furthermore, the system 120 for screening materials for a lithium secondary battery may receive in advance the information necessary to generate the information on the molecular properties and chemical properties of the organic substance. In some embodiments, the information necessary to generate the information on the molecular properties and chemical properties of the organic substance may also be generated using DFT calculations.

The system 120 for screening materials for a lithium secondary battery may generate a material database 122 by storing the information about organic substances based on a plurality of parameters. The material database 122 may store the information aobut the organic substances by dividing it into a plurality of parameters. The plurality of parameters may represent categories for the information on the organic substances. In some embodiments, the plurality of parameters may include parameters associated with an ion type, a molecular weight, a dipole moment, an electron affinity, an ionization energy, a lithium-ion interaction energy, an interaction energy between an organic substance and the anions of a lithium salt, a HOMO energy, a LUMO energy, a protonation energy (PE), a dehydrogenation energy (DE), an interaction energy of oxygen radicals, an electron affinity of a lithium-ion complex containing the organic substance, LUMO of the lithium-ion complex containing the organic substance, an interaction energy between the lithium-ion complex containing the organic substance and the oxygen radicals, an interaction energy between the organic substance and a transition metal, etc.

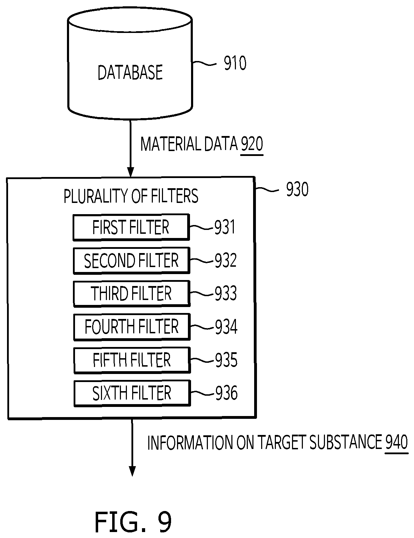

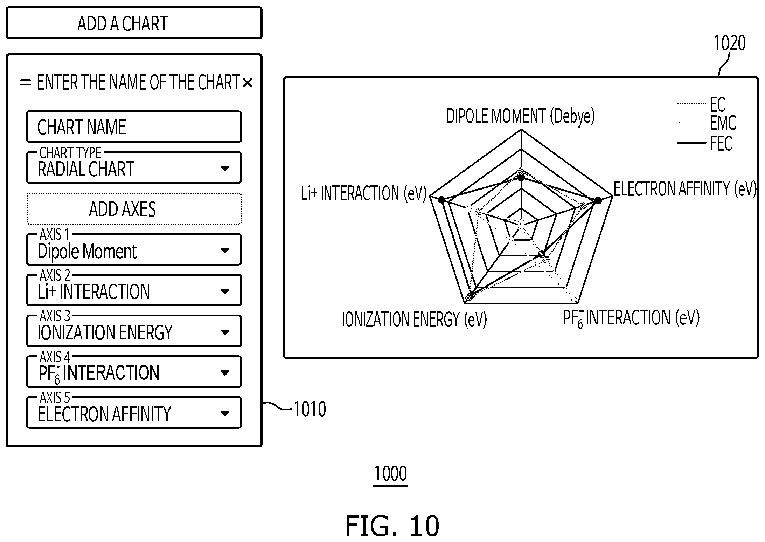

The system 120 for screening materials for a lithium secondary battery may generate output information 130 about a target substance configured to be used in the lithium secondary battery out of the organic substances by applying at least one filters to the material database 122. In a lithium secondary battery containing the target substance, the target substance may form at least a part of a low-resistance SEI (solid electrolyte interphase) layer on the anode of the lithium secondary battery. The system 120 for screening materials for a lithium secondary battery may generate output information 130 about the target substance to a user terminal, etc. Furthermore, the output information 130 about the target substance may be outputted onto a user interface. The schematic of generating output information 130 about the target substance by applying at least one filters to the material database 122, will be described in detail with reference to FIG. 9, and the user interface in which the information 130 about the target substance is outputted onto will be described in detail with reference to FIGS. 10 and. 11.

In some embodiments, when a name that can specify an organic substance is inputted into the system 120 for screening materials for a lithium secondary battery, the system 120 for screening materials for a lithium secondary battery can generate information 110 on the organic substance and automatically build a database (e.g., the material database 122) based on the information 110 on the organic substance.

In some embodiments, organic substances configured to be used in secondary batteries can be readily searched for using the database without any other experiments. It may take a lot of time and effort to find a material that inhibits the deterioration of secondary batteries, but because there is no need to perform repetitive experiments to find a material (e.g., organic substance) that is capable of inhibiting the deterioration of a secondary battery, time, effort, resources, costs, etc., can be saved. In some embodiments, in a lithium secondary battery, an SEI layer can be formed on the anode as the secondary battery is charged and discharged. The secondary battery can be deteriorated due to the electric resistance of the SEI layer. In this manner, by obtaining information on organic substances that form an SEI layer with relatively lower resistance than the SEI layer formed previously, organic substances that slow down or inhibit the deterioration of lithium secondary batteries can be readily searched for.

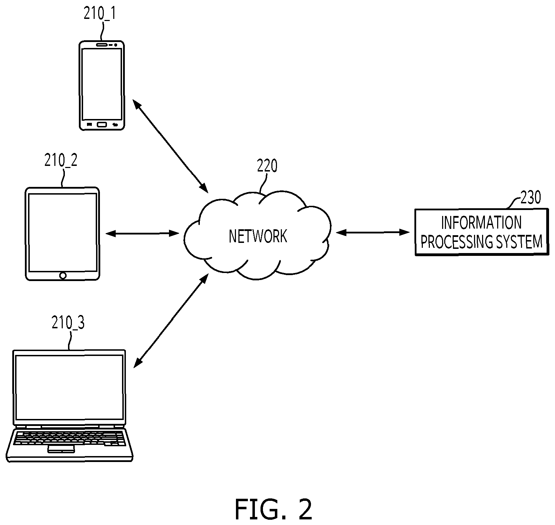

FIG. 2 is a schematic diagram showing a configuration in which an information processing system 230 is communicably connected to a plurality of user terminals 210_1, 210_2, and 210_3 for screening materials for a lithium secondary battery according to embodiments of the present invention. Referring to FIG. 2, the plurality of user terminals 210_1, 210_2, and 210_3 may be connected to the information processing system 230 (e.g., the system 120 for screening materials for a lithium secondary battery in FIG. 1) that can provide a service for screening materials for a lithium secondary battery via a network 220. Here, the plurality of user terminals 210_1, 210_2, and 210_3 may include terminals of users who are recipients of the service for screening materials for a lithium secondary battery.

In one embodiment, the information processing system 230 may include one or more server devices and/or databases, or one or more distributed computing devices and/or distributed databases based on cloud computing services, which can store, provide, and execute computer-executable programs (e.g., downloadable applications) and data associated with providing the service for screening materials for a lithium secondary battery, etc.

The service for screening materials for a lithium secondary battery provided by the information processing system 230 may be provided to users via an application, a web browser, a web browser extension program, etc., for screening materials for a lithium secondary battery installed on each of the plurality of user terminals 210_1, 210_2, and 210_3. For example, the information processing system 230 may provide information or perform processing corresponding to a request for information about a target substance received from the user terminals 210_1, 210_2, and 210_3 via the application for screening materials for a lithium secondary battery, etc.

The plurality of user terminals 210_1, 210_2, and 210_3 may communicate with the information processing system 230 via the network 220. The network 220 may be configured to enable communication between the plurality of user terminals 210_1, 210_2, and 210_3 and the information processing system 230. The network 220 may be formed of, for example, a wired network such as Ethernet, power line communication, a telephone line communication device, and RS-serial communication, a wireless network such as a mobile communication network, WLAN (wireless LAN), Wi-Fi, Bluetooth, and ZigBee, or a combination thereof, depending on the installation environment. The communication method is not limited, and not only a communication method utilizing a communication network that the network 220 may include (as one example, a mobile communication network, wired Internet, wireless Internet, a broadcasting network, a satellite network, etc.), but also short-range wireless communication between the user terminals 210_1, 210_2, and 210_3 may be included.

A mobile phone terminal 210_1, a tablet terminal 210_2, and a PC terminal 210_3 are shown as examples of the user terminals in FIG. 2, but the user terminals are not limited thereto, and the user terminals 210_1, 210_2, and 210_3 may be any computing devices capable of wired and/or wireless communication and capable of having an application, a web browser, etc., for screening materials for a lithium secondary battery installed and executed thereon. In some embodiments, the user terminals may include AI speakers, smartphones, mobile phones, navigation systems, computers, laptops, digital broadcasting terminals, PDAs (personal digital assistants), PMPs (portable multimedia players), tablet PCs, game consoles, wearable devices, IoT (Internet of Things) devices, VR (virtual reality) devices, AR (augmented reality) devices, set-top boxes, etc. Furthermore, FIG. 2 shows that three user terminals 210_1, 210_2, and 210_3 communicate with the information processing system 230 via the network 220, but the present embodiment is not limited thereto, and a configuration may be provided such that a different number of user terminals communicate with the information processing system 230 via the network 220.

FIG. 2 shows a configuration, by way of example, in which a user's request (e.g., a request for information about a target substance) is transferred to the information processing system 230 via the user terminals 210_1, 210_2, and 210_3, but the embodiment is not limited thereto, and the user's request may be provided to the information processing system 230 via an input device associated with the information processing system 230 without going through the user terminals 210_1, 210_2, and 210_3, and the result of processing the user's request (e.g., the information on the target substance 130) may be provided to the user via an output device (e.g., a display, etc.) associated with the information processing system 230.

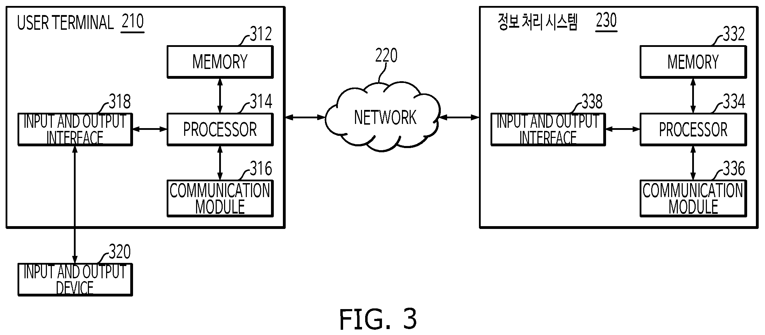

FIG. 3 is a block diagram showing an internal configuration of a user terminal 210 and the information processing system 230 according to embodiments of the present disclosure. The user terminal 210 may refer to any computing device capable of executing applications, web browsers, etc., and capable of wired/wireless communication, and may include, for example, the mobile phone terminal 210_1, the tablet terminal 210_2, the PC terminal 210_3 in FIG. 2. Referring to FIG. 3, the user terminal 210 may include a memory 312, a processor 314, a communication module 316, and an input/output interface 318. Similarly, the information processing system 230 may include a memory 332, a processor 334, a communication module 336, and an input/output interface 338. Referring to FIG. 3, the user terminal 210 and the information processing system 230 may be configured to communicate information and/or data via the network 220 by using the respective communication modules 316 and 336. Further, an input/output device 320 may be configured to input information and/or data to the user terminal 210 or output the information and/or data generated by the user terminal 210 via the input/output interface 318.

The memories 312 and 332 may include any non-transitory computer-readable recording medium. In some embodiments, the memories 312 and 332 may include a permanent mass storage device, such as read-only memory (ROM), a disk drive, a solid-state drive (SSDs), flash memory, etc. In some embodiments, the permanent mass storage device, such as ROM, SSD, flash memory, a disk drive, etc., may be included in the user terminal 210 or the information processing system 230 as a separate persistent storage device distinct from the memories. In some embodiments, the memories 312 and 332 may store an operating system and at least one program code.

These software components may be loaded from computer-readable recording media separate from the memories 312 and 332. Such separate computer-readable recording media may include recording media directly connectable to the user terminal 210 and the information processing system 230, and may include, for example, computer-readable recording media such as floppy drives, disks, tapes, DVD/CD-ROM drives, and memory cards. In some embodiments, the software components may be loaded into the memories 312 and 332 via the communication modules 316 and 336 rather than computer-readable recording media. In some embodiments, at least one program may be loaded onto the memories 312 and 332 based on a computer program installed by files provided via the network 220 by developers or a file distribution system that distributes installation files of an application.

The processors 314 and 334 may be configured to process commands of computer programs by performing basic arithmetic, logic, and input/output operations. The commands may be provided to the processors 314 and 334 by the memories 312 and 332 or the communication modules 316 and 336. In some embodiments, the processors 314 and 334 may be configured to execute the received commands according to program codes stored in recording devices such as the memories 312 and 332.

The communication modules 316 and 336 may provide configurations or functions for the user terminal 210 and the information processing system 230 to communicate with each other via the network 220, and may provide configurations or functions for the user terminal 210 and/or the information processing system 230 to communicate with other user terminals or other systems (as one example, separate cloud systems, etc.). In some embodiments, a request or data (e.g., a request to provide information on a target substance, etc.) generated by the processor 314 of the user terminal 210 according to the program code stored in a recording device such as the memory 312, etc., may be transmitted to the information processing system 230 via the network 220 under the control of the communication module 316. Conversely, control signals or commands provided under the control of the processor 334 of the information processing system 230 may be received by the user terminal 210 via the communication module 316 of the user terminal 210 by way of the communication module 336 and the network 220.

The input/output interface 318 may be a means for interfacing with the input/output device 320. In some embodiments, the input device may include devices such as a camera including an audio sensor and/or an image sensor, a keyboard, a microphone, a mouse, and the output device may include devices such as a display, a speaker, a haptic feedback device, etc. In some embodiments, the input/output interface 318 may be a means for interfacing with a device in which configurations or functions for performing input and output are integrated into one, such as a touchscreen, etc. In some embodiments, when the processor 314 of the user terminal 210 processes a command of a computer program loaded into the memory 312, a service screen, etc., configured using information and/or data provided by the information processing system 230 or another user terminal may be displayed on a display via the input/output interface 318. In FIG. 3, the input/output device 320 is shown not to be included in the user terminal 210 but is not limited thereto and may be formed as a single device together with the user terminal 210. Further, the input/output interface 338 of the information processing system 230 may be a means for interfacing with a device (not shown) for input or output that may be connected to the information processing system 230 or that the information processing system 230 may include. In FIG. 3, the input/output interfaces 318 and 338 are shown as elements configured separately from the processors 314 and 334, but are not limited thereto, and the input/output interfaces 318 and 338 may be configured to be included in the processors 314 and 334.

The user terminal 210 and the information processing system 230 may include additional components other than those shown in FIG. 3. However, it is not necessary to explicitly show most of the prior art components. In one embodiment, the user terminal 210 may be implemented to include at least some of the input/output devices 320 described above. Moreover, the user terminal 210 may further include other components such as a transceiver, a global positioning system (GPS) module, a camera, various sensors, a database, etc. For example, if the user terminal 210 is a smartphone, it may include components that are commonly included in a smartphone, and an implementation may be made such that various components such as, for example, an acceleration sensor, a gyro sensor, a microphone module, a camera module, various physical buttons, buttons using a touch panel, input/output ports, and a vibrator for vibration, all of which can be further included in the user terminal 210.

While a program or application for the service for screening materials for a lithium secondary battery, etc., is running, the processor 314 may receive text, images, videos, voices, and/or actions, etc., entered or selected via the input device such as a touch screen, a keyboard, a camera including an audio sensor and/or an image sensor, and a microphone connected to the input/output interface 318, and may store the received text, images, videos, voices, and/or actions, etc., in the memory 312 or provide any of them to the information processing system 230 via the communication module 316 and the network 220.

The processor 314 of the user terminal 210 may be configured to manage, process, and/or store information and/or data received from the input/output device 320, other user terminals, the information processing system 230, and/or a plurality of external systems. The information and/or data processed by the processor 314 may be provided to the information processing system 230 via the communication module 316 and the network 220. The processor 314 of the user terminal 210 may transmit and output the information and/or data to the input/output device 320 via the input/output interface 318. In some embodiments, the processor 314 may output or display the received information and/or data on a screen associated with the user terminal 210.

The processor 334 of the information processing system 230 may be configured to manage, process, and/or store information and/or data received from the plurality of user terminals 210 and/or a plurality of external systems. The information and/or data processed by the processor 334 may be provided to the user terminal 210 via the communication module 336 and the network 220.

FIGS. 2 and 3 show that the user terminals 210 communicate with the information processing system 230 capable of providing the service for screening materials for a lithium secondary battery via the network, but the present embodiment is not limited thereto, and the user may access or operate the information processing system 230 by using the input/output interface 338 provided by the information processing system 230 without going through the user terminal 210 and the network.

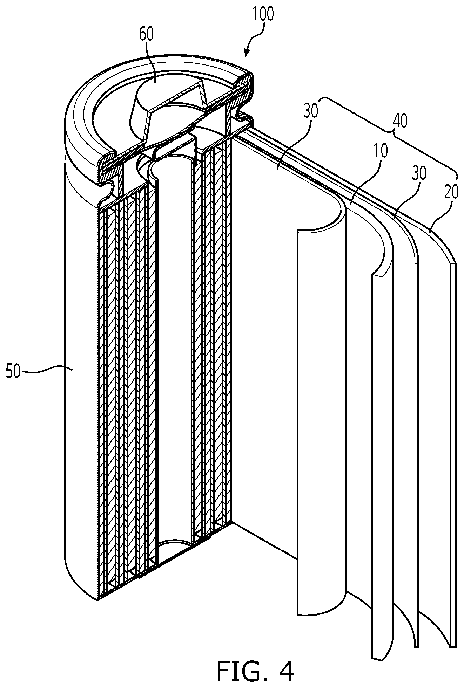

FIG. 4 is a cross-sectional view showing a lithium secondary battery 100 according to embodiments of the present disclosure. Referring to FIG. 4, a lithium secondary battery 100 may include an electrode assembly 40 with a separator 30 interposed between a cathode 10 and an anode 20, and a housing 50 in which the electrode assembly 40 is contained within. The cathode 10, the anode 20, and the separator 30 may be impregnated with an electrolyte (not shown). The lithium secondary battery 100 may include an electrode tab 60 that serves as an electrical path for guiding the electric current formed in the electrode assembly 40 to the exterior of the lithium secondary battery 100. Referring to FIG. 4, the lithium secondary battery is shown to have a cylindrical geometry, but is not limited thereto. In some embodiemnts, the lithium secondary battery may have a prismatic geometry, a pouch geometry, a coin geometry, etc.

The electrolyte solution for a rechargeable lithium battery may include a non-aqueous organic solvent and a lithium salt.

The non-aqueous organic solvent may serve as a medium for transmitting ions taking part in the electrochemical reaction of a battery.

The non-aqueous organic solvent may be a carbonate-based, an ester-based, an ether-based, a ketone-based, or an alcohol-based solvent, an aprotic solvent, or a combination thereof.

The carbonate-based solvent may include dimethyl carbonate (DMC), diethyl carbonate (DEC), dipropyl carbonate (DPC), methylpropyl carbonate (MPC), ethylpropyl carbonate (EPC), methylethyl carbonate (MEC), ethylene carbonate (EC), propylene carbonate (PC), butylene carbonate (BC), and the like.

The ester-based solvent may include methyl acetate, ethyl acetate, n-propyl acetate, dimethyl acetate, methyl propionate, ethyl propionate, decanolide, mevalonolactone, valerolactone, caprolactone, and the like.

The ether-based solvent may include dibutyl ether, tetraglyme, diglyme, dimethoxyethane, 2-methyltetrahydrofuran, 2,5-dimethyltetrahydrofuran, tetrahydrofuran, and the like.

The ketone-based solvent may include cyclohexanone, and the like.

The alcohol-based solvent may include ethanol, isopropyl alcohol, and the like.

The aprotic solvent may include nitriles such as R-CN (wherein R is a C2 to C20 linear, branched, or cyclic hydrocarbon group, which may include a double bond, an aromatic ring, or an ether bond, and the like; amides such as dimethylformamide; dioxolanes such as 1,3-dioxolane, 1,4-dioxolane, and the like; sulfolanes, and the like; etc.

The non-aqueous organic solvents may be used alone or in combination with two or more.

In addition, when using a carbonate-based solvent, a cyclic carbonate and a chain carbonate may be combined and used, and the cyclic carbonate and the chain carbonate may be combined in a volume ratio of about 1:1 to about 1:9.

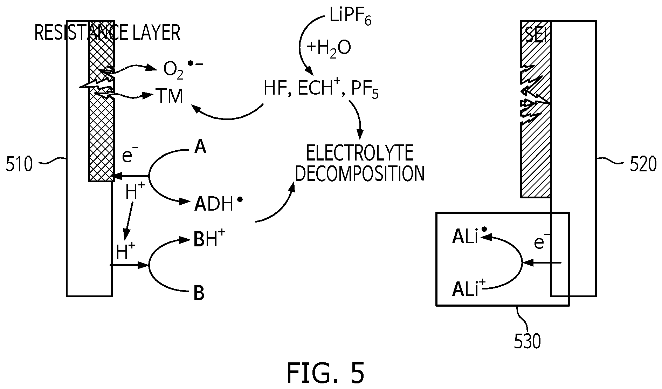

FIG. 5 shows a cathode 510, an anode 520, and an electrolyte according to embodiments of the present disclosure. FIG. 5 may show factors (e.g., reaction formulas) that cause the deterioration of a lithium secondary battery.

Referring to the cathode 510 shown in FIG. 5, an interfacial film (a resistance layer as shown in FIG. 5) may be formed on the cathode 510. For example, a cathode electrolyte interphase (CEI) layer may be formed on the interface of the cathode 510. The interfacial film may have resistance. Accordingly, the secondary battery may be deteriorated by the resistance of the interfacial film.

The interfacial film formed on the cathode 510 may have cracks through which the cathode 510 is exposed. Oxygen radicals (O₂^{·-} as shown in FIG. 5) may be generated by the cathode 510 exposed through the cracks in the interfacial film, and transition metal ions (TM (transition metal) as shown in FIG. 5) may be generated. For example, oxygen radicals and transition metal ions may be generated as the cathode 510 reacts with the electrolyte. The oxygen radicals and transition metal ions may cause side reactions inside the secondary battery, thereby deteriorating the secondary battery.

A first material (A as shown in FIG. 5) contained in the electrolyte may be oxidized at or around the cathode 510 and a dehydrogenation reaction may occur. The hydrogen ions generated by the dehydrogenation reaction may react with a second material (B as shown in FIG. 5) contained in the electrolyte, causing a protonation reaction. The dehydrogenated first material (ADH^{·} as shownin FIG. 5) and the protonated second material (BH⁺ as shown in FIG. 5) can cause electrolyte decomposition. Additional dehydrogenated first material and additional protonated second material are generated as the secondary battery is repetitively being charged and discharged, and the dehydrogenated first material and the protonated second material can further decompose the electrolyte. As the electrolyte, allowing lithium to migrate decomposes, the secondary battery can deteriorate.

Referring to the anode 520 shown in FIG. 5, a solid electrolyte interphase (SEI) layer may be formed on the interface of the anode 520. Further, a reduction reaction may occur in a lithium-ion complex (ALi⁺ as shown in FIG. 5) in which another material contained in the electrolyte is bonded with lithium ions at or around the anode 520. A reduced lithium-ion complex (ALi^{·} as shown in FIG. 5) may be generated by the reduction reaction. The anode 520 may deteriorate by taking away electrons around the anode 520 by the reduction reaction.

The deterioration factors related to the electrolyte will be described based on an example where that the cathode 510 of the lithium secondary battery contains lithium hexafluorophosphate (LiPF₆) and the electrolyte contains ethylene carbonate (EC), which is an organic solvent of the lithium secondary battery. Referring to the electrolyte, lithium hexafluorophosphate may react with water, producing HF, PFs, ECH⁺ (protonated ethylene carbonate), etc. At least some of the products may react with transition metal ions or cause electrolyte decomposition. Further, ECH⁺ may react with EC and H₂O, and EC may be decomposed into acetylenediol (HOCCOH) and CO₂. As the secondary battery is used continuously, EC and electrolyte decomposition may continue to occur through the processes described above, causing the secondary battery to deteriorate.

Among the deterioration factors of the secondary battery, as the resistance of the SEI layer in the formation of the SEI layer increases, the secondary battery may further deteriorate. Referring to FIG. 5, it may be necessary to search for a material that can inhibit deterioration related to the main deterioration reaction formula 530. In some embodiments, in the main deterioration reaction formula 530, as it becomes more difficult for the organic substance contained in the electrolyte to react with the lithium ions than a representative organic solvent (e.g., EC) that is an organic solvent of the lithium secondary battery, it becomes more likely that the organic substance may be involved in the formation of the SEI layer. In some embodiments, in the main deterioration reaction formula 530, as it becomes easier for the organic substance contained in the electrolyte to react with the lithium ions than a representative additive (e.g., FEC) that is an electrolyte additive of the lithium secondary battery, it becomes more likely that the representative additive may be involved in the formation of the SEI layer. As a result, the representative additive is involved first in the formation of the SEI layer, the SEI layer of the lithium secondary battery containing an organic substance that can be involved next in the formation of the SEI layer has a low resistance characteristic, and deterioration of the secondary battery can be inhibited. The system for screening materials for a lithium secondary battery (e.g., the system 120 for screening materials for a lithium secondary battery as shown in FIG. 1) can output information on a target substance (e.g., the information 130 on the target substance as shown in FIG. 1) that is an organic substance for inhibiting the deterioration associated with the main deterioration reaction formula 530 by applying at least one filters to the database (e.g., the material database 122 as shown in FIG. 1).

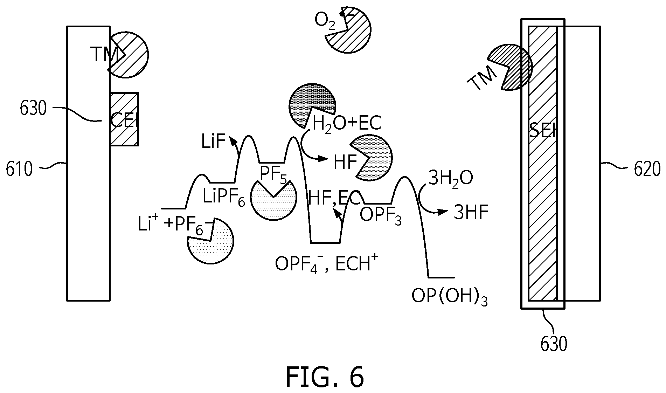

FIG. 6 shows a cathode 610, an anode 620, and an electrolyte according to embodiments of the present disclosure.

Referring to the cathode 610 shown in FIG. 6, a CEI layer may be formed on the interface of the cathode 610. In some embodiments, an organic substance contained in the electrolyte may be oxidized at or around the cathode 610, thereby forming a CEI layer.

Transition metal (TM as shown in FIG. 6) ions that deteriorate the secondary battery at or around the cathode 610 may bond with the organic substance contained in the electrolyte. As a result, the deterioration of the secondary battery can be inhibited.

Referring to the anode 620 shown in FIG. 6, an SEI layer may be formed on the interface of the anode 620. In some embodiments, the organic substance contained in the electrolyte may be oxidized at or around the anode 620, thereby forming an SEI layer.

Transition metal ions that deteriorate the secondary battery at or around the anode 620 may bond with the organic substance contained in the electrolyte. As a result, the deterioration of the secondary battery can be inhibited.

The electrolyte may contain oxygen radicals generated within the secondary battery. The oxygen radicals that contributed to the deterioration of the secondary battery may bond with the organic substance contained in the electrolyte. As a result, the deterioration of the secondary battery can be inhibited.

The lithium salt contained in the electrolyte may cause deterioration of the secondary battery through various reaction pathways. The deterioration factors related to the electrolyte will be described based on an example where the cathode 610 of the lithium secondary battery contains lithium hexafluorophosphate (LiPF₆) and the electrolyte contains ethylene carbonate (EC), which is an organic solvent of the lithium secondary battery. The electrolyte may contain lithium hexafluorophosphate, which is a lithium salt, and may contain lithium ions and hexafluorophosphate ions. The hexafluorophosphate ions may bond with the organic substance contained in the electrolyte. Phosphorus pentafluoride (PFs) present on the reaction pathway of lithium hexafluorophosphate may bond with the organic substance contained in the electrolyte. Water, which may be involved in the reaction pathway of lithium hexafluorophosphate may bond with the organic substance contained in the electrolyte. Hydrogen fluoride (HF), which may be involved in the reaction pathway of lithium hexafluorophosphate, may bond with the organic substance contained in the electrolyte. As a result, the deterioration of the secondary battery may be inhibited.

In the formation of the SEI layer, the secondary battery may deteriorate if the organic solvent that helps the lithium ions migrate forms the SEI layer. Referring to FIG. 6, it may be necessary to search for an organic substance that can form the SEI layer in place of the organic solvent in the main role 630 of the organic substance. In some embodiments, if the LUMO of a lithium-ion complex containing an organic substance is less than the LUMO of a lithium-ion complex containing a representative organic solvent, the organic substance can form at least a part of the SEI layer. Further, as it becomes easier for the representative organic solvent (e.g., EC), which is an organic solvent of the lithium secondary battery, to react with the lithium ions than the organic substance contained in the electrolyte, the deterioration of the secondary battery containing the electrolyte can further be inhibited. The system for screening materials for a lithium secondary battery (e.g., the system 120 for screening materials for a lithium secondary battery as shown in FIG. 1) can output information on a target substance (e.g., the information 130 on the target substance as shown in FIG. 1) that is an organic substance performing the main role 630 of the organic substance by applying at least one filters to the database (e.g., the material database 122 as shown in FIG. 1).

FIG. 7 and Table 1 show example information about organic substances according to embodiments of the present disclosure. The system for screening materials for a lithium secondary battery may generate a database by receiving information about organic substances and storing the information on the organic substances (e.g., the information 110 about the organic substance as shown in FIG. 1) based on a plurality of parameters.

**Table 1**

| Structure | NAME | SMILES | Type | Mass | Electron Affinity | Ionization Energy | Ni²+ Interaction |
|---|---|---|---|---|---|---|---|
| 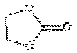 | EC | C1COC(=O)O1 | Neutral | 88.06 | -0.95 | 11.12 | -1.86 |
| 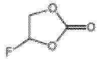 | FEC | C1C(OC(=O)O1)F | Neutral | 106.05 | -0.87 | 11.1 | -1.65 |
| 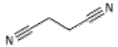 | SN | N#CCCC#N | Neutral | 80.09 | -0.5 | 12.04 | -7.4 |
| 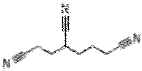 | HTCN | N#CCCC(C#N)CCCC#N | Neutral | 161.2 | -0.48 | 11.19 | -10.8 |
| 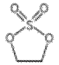 | DTD | C1COS(=O)(=O)O1 | Neutral | 124.12 | -0.79 | 11.18 | -6.35 |
| 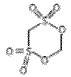 | MMDS | O=S1(=O)CS(=O)(=O)OCO1 | Neutral | 188.18 | 1.05 | 11.77 | -7.01 |
| 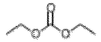 | DEC | CCOC(=O)OCC | Neutral | 118.13 | -1.28 | 10.36 | -7.22 |
| 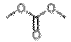 | DMC | COC(=O)OC | Neutral | 90.08 | -1.48 | 10.85 | -6.57 |
| 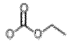 | EA | CCOC(=O)C | Neutral | 88.11 | -1.29 | 10.04 | -0.1 |
| 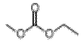 | EMC | CCOC(=O)OC | Neutral | 104.1 | -1.3 | 10.39 | -6.92 |
| 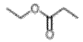 | EP | CCC(=O)OCC | Neutral | 102.13 | -1.25 | 9.94 | -7.08 |
| 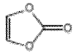 | VC | C1=COC(=O)O1 | Neutral | 86.05 | -1.14 | 9.69 | -6.3 |

Referring to FIG. 7 and Table 1, the organic substances may include ethylene carbonate (EC), fluoroethylene carbonate (FEC), propylene carbonate (PC), vinyl ethylene carbonate (VEC), propene sultone (PS), propane sultone (PES), diethyl carbonate (DEC), dimethyl carbonate (DMC), ethyl acetate (EA), ethyl methyl carbonate (EMC), ethyl propionate (EP), vinylene carbonate (VC), etc. The information on the organic substance may include the name, SMILES, etc., of a compound as a name that can specify the organic substance. The information on the organic substance may include information corresponding to the molecular structure (Structure as shown in FIG. 7 and Table 1), ion type of the organic substance (Type as shown in FIG. 7 and Table 1), molecular weight (Mass as shown in FIG. 7 and Table 1), electron affinity (Electron Affinity as shown in FIG. 7 and Table 1), ionization energy (Ionization Energy as shown in FIG. 7 and Table 1), lithium-ion interaction energy (Li⁺ Interaction as shown in FIG. 7 and Table 1), and interaction energy between the organic substance and hexafluorophosphate ions (PF₆⁻ Interaction as shown in FIG. 7 and Table 1). Here, the unit of energy may be eV.

The plurality of parameters may include parameters associated with each of the name, SMILES, ion type, molecular weight, electron affinity, ionization energy, and lithium-ion interaction energy of a compound, and the interaction energy between the organic substance and hexafluorophosphate ions. However, without being limited thereto, the plurality of parameters may include more or fewer parameters than the parameters shown in FIG. 7 and Table 1, the organic substances may include more or fewer organic substances than the organic substances shown in FIG. 7 and Table 1, and the information on the organic substances may include information corresponding to more or fewer plurality of parameters.

The system for screening materials for a lithium secondary battery may receive (or generate) information on the organic substances described above, and generate a database based on the information on the organic substances.

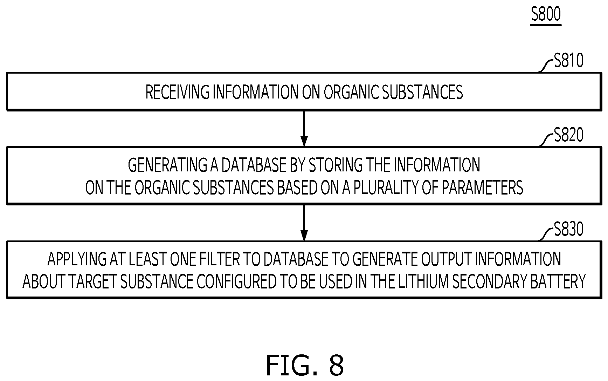

FIG. 8 is a flowchart showing an example method S800 for screening materials for a lithium secondary battery according to one embodiment of the present disclosure. The method S800 for screening materials for a lithium secondary battery may be performed by a system for screening materials for a lithium secondary battery (e.g., the system 120 for screening materials for a lithium secondary battery as shown in FIG. 1). The system for screening materials for a lithium secondary battery may include a processor.

The method S800 for screening materials for a lithium secondary battery may begin by receiving information on organic substances (S810). In one embodiment, the processor may generate a database by storing the information on the organic substances based on a plurality of parameters (S820). For example, the plurality of parameters may include parameters associated with at least one of the ion type, molecular weight, electron affinity, ionization energy, lithium-ion (Li⁺) interaction energy, LUMO (lowest unoccupied molecular orbital) energy, LUMO energy of a lithium-ion complex containing an organic substance, or electron affinity of the lithium-ion complex containing the organic substance.

In some embodiments, the processor may generate output information on a target substance to be used in the lithium secondary battery among the organic substances by applying at least one filters to the database (S830). Here, the target substance may form at least a part of a low-resistance SEI layer on the anode of the lithium secondary battery.

In some embodiments, the at least one filters may include a first filter, and the first filter may filter out materials for which the ionization energy of a material included in the database is greater than a predetermined ionization energy threshold, out of materials included in the database. The predetermined ionization energy threshold may be associated with the ionization energy of a representative organic solvent, which is an organic solvent of the lithium secondary battery. In some embodiments, the representative organic solvent may be one of ethylene carbonate (EC), fluoroethylene carbonate (FEC), ethyl methyl carbonate (EMC), dimethyl carbonate (DMC), ethyl propionate (EP), or ethyl acetate (EA).

In some embodiments, the at least one filters may include a second filter, and the second filter may filter out materials for which the lithium-ion interaction energy of a material included in the database is greater than a predetermined lithium-ion interaction energy threshold, out of the materials included in the database. The predetermined lithium-ion interaction energy threshold may be associated with the lithium-ion interaction energy of the representative organic solvent, which is the organic solvent of the lithium secondary battery.

In some embodiments, the at least one filters may include a third filter, and the third filter may filter out materials for which the lithium-ion interaction energy of a material included in the database is less than a predetermined lithium-ion interaction energy threshold, out of the materials included in the database. The predetermined lithium-ion interaction energy threshold may be associated with the interaction energy between a representative additive, which is an electrolyte additive of the lithium secondary battery, and lithium ions.

In some embodiments, the at least one filters may include a fourth filter, and the fourth filter may filter out materials for which the LUMO energy of a lithium-ion complex containing a material included in the database is less than a predetermined LUMO energy threshold, out of the materials included in the database. The predetermined LUMO energy threshold may be associated with the LUMO energy of a representative lithium-ion complex containing the representative organic solvent, which is the organic solvent of the lithium secondary battery.

In some embodiments, the at least one filters may include a fifth filter, the fifth filter may filter out materials for which the electron affinity of a lithium-ion complex containing a material included in the database is greater than a predetermined electron affinity threshold, out of the materials included in the database. The predetermined electron affinity threshold may be associated with the electron affinity threshold of the representative lithium-ion complex containing the representative organic solvent, which is the organic solvent of the lithium secondary battery.

In some embodiments, the processor may receive an input that selects at least one of the plurality of parameters. The processor may receive information on a numerical range for the selected parameter. The at least one filters may include a filter, and the filter may filter out materials for which information associated with at least one of the selected plurality of parameters of a material included in the database falls within the numerical range, out of the materials included in the database.

In some embodiments, the processor may receive an input that has selected at least one of the plurality of parameters on a user interface. The processor may output information on at least one of the plurality of parameters selected from the information on the target substance onto the user interface.

The flowchart in FIG. 8 and the foregoing description are merely one example of the present disclosure, and the scope of the present disclosure is not limited to the flowchart in FIG. 8 and the foregoing description. For example, one or more steps in the flowchart and the foregoing description may be added/modified/deleted, the order of one or more steps may be changed, and one or more steps may be performed simultaneously.

The methods described above may be provided as computer programs stored on a computer-readable recording medium for execution on a computer. The medium may continue to store computer-executable programs or temporarily store them for execution or download. Further, the medium may be a variety of recording or storage means in the form of a single piece of hardware or a combination of several pieces of hardware, and is not limited to media directly connected to a computer system but may be distributed over a network as well. Examples of media may be those configured to store program instructions, including magnetic media such as hard disks, floppy disks, and magnetic tapes, optical recording media such as CD-ROMs and DVDs, magneto-optical media such as floptical disks, ROM, RAM, flash memory, etc. Furthermore, examples of other media may include recording or storage media managed by app stores that distribute applications, sites that supply or distribute various other software, servers, etc.

The methods, operations, or techniques of the present disclosure may be implemented by a variety of means. For example, these techniques may be implemented in hardware, firmware, software, or combinations thereof. Those having ordinary skill in the art will appreciate that the various example logic blocks, modules, circuits, and algorithm steps described in connection with the disclosure herein may be implemented in electronic hardware, computer software, or a combination of both. To clearly describe this interchangeability of hardware and software, the various example components, blocks, modules, circuits, and steps have been described above generally in terms of their functionality. Whether such functionality is implemented in hardware or software depends on the particular application and design requirements imposed on the overall system. Those having ordinary skill in the art may implement the described functionality in a variety of ways for each particular application, but such implementations should not be construed as departing from the scope of the present disclosure.

In hardware implementation, the processing units used to perform the techniques may be implemented within one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described in the present disclosure, computers, or combinations thereof.

Therefore, the various example logic blocks, modules, and circuits described in connection with the present disclosure may be implemented or performed in any combination of general-purpose processors, DSPs, ASICs, FPGAs or other programmable logic devices, discrete gate or transistor logic, discrete hardware components, or those designed to perform the functions described herein. The general-purpose processor may be a microprocessor, but in another example, the processor may be any conventional processor, controller, microcontroller, or state machine. The processor may also be implemented as a combination of computing devices, for example, a combination of a DSP and a microprocessor, a plurality of microprocessors, one or more microprocessors in conjunction with a DSP core, or any other configurations.

In firmware and/or software implementation, the techniques may be implemented as instructions stored on a computer-readable medium such as random-access memory (RAM), read-only memory (ROM), non-volatile random-access memory (NVRAM), PROM (programmable read-only memory), EPROM (erasable programmable read-only memory), EEPROM (electrically erasable PROM), flash memory, compact discs (CDs), magnetic or optical data storage devices, etc. The instructions may be executable by one or more processors, and may cause the processor(s) to perform certain aspects of the functionality described in the present disclosure.

If implemented in software, the techniques described above may be stored on or transmitted via a computer-readable medium, as one or more instructions or code. The computer-readable media may include computer storage media and communication media, including any media that facilitate the transmission of a computer program from one place to another. The storage media may be any available media that can be accessed by a computer. By way of non-limiting example, the computer-readable media may include RAM, ROM, EEPROM, CD-ROM or other optical disc storages, magnetic disk storage or other magnetic storage devices, or any other media that can be used to transport or store the desired program code in the form of instructions or data structures and that can be accessed by a computer. Moreover, any access is appropriately made to a computer-readable medium.

For example, if the software is transmitted from websites, servers, or other remote sources using coaxial cables, fiber optic cables, twisted pair cables, digital subscriber lines (DSLs), or wireless technologies such as infrared, radio, and microwave, then the coaxial cables, fiber optic cables, twisted pair cables, digital subscriber lines, or wireless technologies such as infrared, radio, and microwave fall within the definition of media. The disks and discs used herein include CDs, laser discs, optical discs, digital versatile discs (DVDs), floppy disks, and Blu-ray discs, wherein the disks typically reproduce data magnetically, whereas the discs reproduce data optically using lasers. Combinations of the above should also fall within the scope of computer-readable media.

The software modules may reside in RAM memory, flash memory, ROM memory, EPROM memory, EEPROM memory, registers, a hard disk, a removable disk, a CD-ROM, or any other form of storage medium known. An example storage medium may be connected to the processor such that the processor can read information from or write information to the storage medium. In another example, the storage medium may be integrated into the processor. The processor and storage medium may be present within an ASIC. The ASIC may be present within a user terminal. In another example, the processor and storage medium may be present as separate components in a user terminal.

Although the embodiments have been described above as utilizing aspects of the presently disclosed subject matter in one or more standalone computer systems, the present disclosure is not limited thereto but may be implemented in conjunction with any computing environment, such as a network or distributed computing environment. Furthermore, aspects of the subject matter in the present disclosure may be implemented in a plurality of processing chips or devices, and storage may be affected similarly across the plurality of devices. These devices may include PCs, network servers, and portable devices.

FIG. 9 shows generating output information 940 about a target substance using a plurality of filters 930 according to embodiments of the present disclosure. A system for screening materials for a lithium secondary battery may include a database 910 and a plurality of filters 930. The system for screening materials for a lithium secondary battery may generate output information 940 about a target substance by applying at least one filters included in the plurality of filters 930 to data included in the database 910. Here, the lithium secondary battery containing the target substance can inhibit the deterioration of the lithium secondary battery caused by the anions of a lithium salt by the target substance.

The database 910 may include information on organic substances. For example, the database 910 may include a name capable of specifying an organic substance, molecular properties and chemical properties of the organic substance, etc. Further, the database 910 may include information necessary to generate information on the molecular properties and chemical properties of the organic substance. The information necessary to generate the information on the molecular properties and chemical properties of the organic substance may be determined in advance, or received in advance before applying the plurality of filters 930.

Material data 920 may be filtered by having at least some of the plurality of filters 930 applied thereto, thereby generating output information 940 about the target substance. The material data 920 may be at least part of the data included in the database 910. In some embodiments, by having a first filter 931, a second filter 932, a third filter 933, and a fourth filter 934 applied to the material data 920, the information 940 about the target substance may be generated. In some embodiments, by having a fifth filter 935 applied to material data 920, the information 940 about the target substance may be generated. Filtering may be performed on materials.

The plurality of filters 930 may include the first filter 931 to the sixth filter 936. Referring to FIG. 9, six filters are shown, but the present disclosure is not limited thereto. In some embodiments, the plurality of filters 930 may include fewer or more filters than six filters.

In some embodiments, the first filter 931 may filter out materials for which the ionization energy of a material included in the material data 920 is greater than an ionization energy threshold (e.g., a predetermined ionization energy threshold). The ionization energy threshold may be associated with the ionization energy of a representative organic solvent, which is an organic solvent of the lithium secondary battery. In some embodiments, the representative organic solvent may be one of ethylene carbonate (EC), fluoroethylene carbonate (FEC), ethyl methyl carbonate (EMC), dimethyl carbonate (DMC), ethyl propionate (EP), or ethyl acetate (EA). The electron affinity threshold may be a value obtained by adding or subtracting a value of 0.1 eV or less to or from the electron affinity of the representative organic solvent. In some embodiments, if the representative organic solvent is DMC, the electron affinity of the representative organic solvent may be 10.85 eV. In this case, the electron affinity threshold may be one of 10.75 eV to 10.95 eV.

In some embodiments, the second filter 932 may filter out materials for which the lithium-ion interaction energy of a material included in the material data 920 is greater than a first lithium-ion interaction energy threshold (e.g., a predetermined first lithium-ion interaction energy threshold). The first lithium-ion interaction energy threshold may be associated with the lithium-ion interaction energy of the representative organic solvent. Specifically, the first lithium-ion interaction energy threshold may be a value obtained by adding or subtracting a value of 0.1 eV or less to or from the lithium-ion interaction energy of the representative organic solvent. In some embodiments, if the representative organic solvent is EC, the lithium-ion interaction energy of the representative organic solvent may be -1.86 eV. In this case, the first lithium-ion interaction energy threshold may be one of -1.96 eV to -1.76 eV.

In some embodiments, the third filter 933 may filter out materials for which the lithium-ion interaction energy of a material included in the material data 920 is less than a second lithium-ion interaction energy threshold (e.g., a predetermined second lithium-ion interaction energy threshold). The second lithium-ion interaction energy threshold may be associated with the interaction energy between a representative additive, which is an electrolyte additive of the lithium secondary battery, and lithium ions. In some embodiments, the representative additive may be one of vinylene carbonate (VC) and fluoroethylene carbonate (FEC). The lithium-ion interaction energy threshold may be a value obtained by adding or subtracting a value of 0.1 eV or less to or from the lithium-ion interaction energy of the representative additive. In some embodiments, if the representative additive is FEC, the lithium-ion interaction energy of the representative additive may be -1.65 eV. In this case, the lithium-ion interaction energy threshold may be one of -1.75 eV to -1.55 eV.

In some embodiments, the fourth filter 934 may filter out materials for which the LUMO energy of a lithium-ion complex containing a material included in the material data 920 is less than a LUMO energy threshold (e.g., a predetermined LUMO energy threshold). The LUMO energy threshold may be associated with the LUMO energy of a representative lithium-ion complex containing the representative organic solvent. Specifically, the LUMO energy threshold may be a value obtained by adding or subtracting a value of 0.1 eV or less to or from the LUMO energy of the representative lithium-ion complex. In some embodiments, if the representative organic solvent is EC, the LUMO energy of the representative lithium-ion complex may be -4.89 eV. In this case, the LUMO energy threshold may be one of -4.99 eV to-4.79 eV.

In some embodiments, the fifth filter 935 may filter out materials for which the electron affinity of a lithium-ion complex containing a material included in the material data 920 is greater than an electron affinity threshold (e.g., a predetermined electron affinity threshold). The electron affinity threshold may be associated with the electron affinity of the representative lithium-ion complex containing the representative organic solvent. Specifically, the electron affinity threshold may be a value obtained by adding or subtracting a value of 0.1 eV or less to or from the electron affinity energy of the representative lithium-ion complex.

In some embodiments, the system for screening materials for a lithium secondary battery may receive a first input that selects at least one of a plurality of parameters. Moreover, the system for screening materials for a lithium secondary battery may receive information about a first numerical range for the selected parameter (or parameters). The system for screening materials for a lithium secondary battery may generate a sixth filter 936 based on the first input that selects at least one of the plurality of parameters and the information about the first numerical range. In response to the information on the parameter selected by the first input out of the information about the materials included in the material data 920 falling within the first numerical range, the sixth filter 936 may filter out the corresponding material.

In some embodiments, the system for screening materials for a lithium secondary battery may receive a second input that selects at least one of the plurality of parameters. Further, the system for screening materials for a lithium secondary battery may receive information about a second numerical range for the selected parameter (or parameters). After the sixth filter 936 is generated, the system for screening materials for a lithium secondary battery may further generate a seventh filter based on the second input that selects at least one of the plurality of parameters and the information about the second numerical range. Then, at least one of the sixth filter 936 or the seventh filter may be applied to the material data 920. In some embodiments, a plurality of filters may be generated by the input of the user, and the plurality of filters generated by the input of the user may be applied to the material data 920.

In some embodiments, the filter to be applied to the material data 920 may be determined in advance before filtering is performed. In some embodiments, the system for screening materials for a lithium secondary battery may receive an input that selects a filter for performing filtering. The system for screening materials for a lithium secondary battery may determine a filter to apply to the material data 920 based on the input that selects a filter. In some embodiments, the system for screening materials for a lithium secondary battery may receive an input that selects the second filter 932 to the fourth filter 934, and determine the second filter 932 to the fourth filter 934 as filters to apply to the material data 920.

In some embodiments, if at least two of the plurality of filters 930 are applied to the material data 920, "AND" or "OR" logic may be applied. In some embodiments, if the first filter 931 and the second filter 932 are applied to the material data 920, the material filtered by the first filter 931 and filtered by the second filter 932 may be determined as the target substance. In some embodiments, if the first filter 931 and the second filter 932 are applied to the material data 920, the material filtered by the first filter 931 and the material filtered by the second filter 932 may be determined as the target substances. Whether to apply "AND" or "OR" logic may be determined in advance, or information associated therewith may be received (e.g., an input may be received via a user interface).

In some embodiments, the system for screening materials for a lithium secondary battery may determine the filtered material as the target substance. The system for screening materials for a lithium secondary battery may output information about the filtered material as information 940 about the target substance. In some embodiments, the information 940 on the target substance may be outputted to a user terminal, etc., and the information 940 on the target substance may be outputted onto a user interface of the user terminal.

In some embodiments, the system for screening materials for a lithium secondary battery may generate output information 940 about the target substance by applying the first filter 931 to the fourth filter 934 to the material data 920. For example, the materials filtered by applying the first filter to the fourth filter 934 may be DMC, EC, FEC, VC, etc. The system for screening materials for a lithium secondary battery may generate output information about DMC, EC, FEC, VC, etc., as the information 940 about the target substance.

In some embodiments, organic substances that form at least a part of the low-resistance SEI layer on the anode of the lithium secondary battery can be screened using at least some of the plurality of filters 930. In some embodiments, organic substances having oxidation stability in the lithium secondary battery containing the representative organic solvent can be filtered out using the first filter 931. Organic substances that form an SEI layer prior to the representative organic solvent can be filtered out using the second filter 932. Organic substances that allow the representative additive to form an SEI layer with more priority can be filtered out using the third filter 933. Organic substances capable of forming an SEI layer on the anode in the lithium secondary battery can be filtered out using the fourth filter 934. Organic substances that cause the lithium-ion complex containing an organic substance to be reduced more easily than the representative lithium-ion complex containing the representative organic solvent can be filtered out using the fifth filter 935. Moreover, by allowing the user to select at least one of the plurality of parameters and to input a numerical range for the selected parameter (or parameters) to generate a filter (e.g., the sixth filter 936), suitable organic substances that can further inhibit the deterioration of the lithium secondary battery can be searched for.

FIG. 10 shows an example a user interface 1000 having output information about target substances, according to embodiments of the present disclosure. A user interface 1000 may generate output information about target substances. Referring to FIG. 10, the user interface 1000 may include a graphical interface 1020 in which the information on the target substances can be displayed. In some embodiments, the target substances may include EC, EMC, and FEC. The graphical interface 1020 may display graphs corresponding to each of EC, EMC, and FEC.

In some embodiments, the graphcal interface 1020 may display the information on the target substances. In some embodiments, the graphical interface 1020 may display information corresponding to an electric dipole (Dipole Moment on the graphical interface 1020 as shown in FIG. 10), an electron affinity (Electron Affinity on the graphical interface 1020 as shown in FIG. 10), a hexafluorophosphate ion interaction energy (PF₆⁻ Interaction on the graphical interface 1020 as shown in FIG. 10), an ionization energy (Ionization Energy on the graphical interface 1020 as shown in FIG. 10), and a lithium-ion interaction energy (Li⁺ Interaction on the graphical interface 1020 as shown in FIG. 10), among the information on the target substances. However, the type of information on the target substances is not limited thereto, and at least some of the information on the target substances may be displayed.

In some embodiments, the type of information displayed on the graphical interface 1020 may be determined through the input received via a graphical control interface 1010. The type of information displayed may correspond to at least some of a plurality of parameters (e.g., the plurality of parameters that served as the basis for generating the database). In some embodiments, by an input onto the graphical control interface 1010, an electric dipole, a lithium-ion interaction energy, an ionization energy, a hexafluorophosphate ion interaction energy, and an electron affinity may be determined as the types of information displayed. Further, the type of graph (e.g., a radial chart, a bar chart, etc.) may be determined via the graphical control interface 1010.

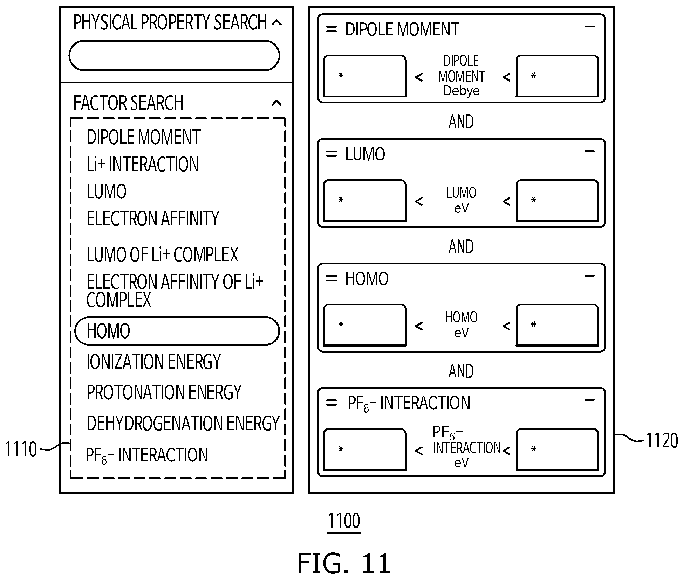

FIG. 11 shows an example filter input interface 1100 according to embodiments of the present disclosure. The system for screening materials for a lithium secondary battery may generate a filter (e.g., the fifth filter 935 in FIG. 9) based on an input received via the filter input interface 1100.

In some embodiments, the filter input interface 1100 may include a parameter selection interface 1110 and a numeric input interface 1120. At least some of a plurality of parameters (e.g., the plurality of parameters that served as the basis for generating the database) may be selected via the parameter selection interface 1110. In some embodiments, the filter input interface 1100 may further include an input window for searching for parameters in order to select at least some of the plurality of parameters.

In some embodiments, the numeric input interface 1120 may display an input window associated with the selected parameter (or parameters). Referring to FIG. 11, the dipole moment, LUMO, HOMO, and hexafluorophosphate ion interaction energy may be selected, and the corresponding parameters may be displayed on the numeric input interface 1120. Numerical ranges for the corresponding parameters may be entered via the numeric input interface 1120. However, if there is an parameter (e.g., an ion type) that is not related to a numerical value among the plurality of parameters and the corresponding parameter is selected, the corresponding selected parameter may not be displayed on the numeric input interface 1120, or the input window for the corresponding selected parameter may be disabled.

In some embodiments, the system for screening materials for a lithium secondary battery may generate a plurality of filters based on the information on the selected parameter (or parameters) and the numerical range. In some embodiments, a first filter may be generated based on information on a selected first parameter and a first numerical range for the first parameter. Moreover, a second filter may be generated based on information on a selected second parameter and a second numerical range for the second parameter. The first filter and the second filter may be subject to the application of "AND" or "OR" logic. In some embodiments, the material filtered by the first filter and the second filter may be generated as output including a target substance. In some embodiments, the material filtered by the first filter and the material filtered by the second filter may be determined as target substances.

As described with reference to FIGS. 10 and 11, filters can be readily generated via the user interface, and target substances capable of inhibiting the deterioration of the lithium secondary battery can be readily searched for using the generated filters. The information about the target substances can be displayed with high readability via the user interface. The user can compare the substances included in the target substances with each other via the user interface, and more suitable substances can be selected.

Although the present disclosure has been described with respect to embodiments thereof, the present disclosure is not limited thereto. Various modifications and variations can be made thereto by those skilled in the art within the spirit of the present disclosure.

### DESCRIPTION OF NOTABLE REFERENCE SYMBOLS

- 110:: Information on organic substances
- 120:: System for screening materials for a lithium secondary battery
- 122:: Material database
- 124:: Information on target substances

## Claims

1. A method of screening materials configured to be used in a lithium secondary battery (100), wherein the method is preferably a computer-implemented method, comprising:
receiving information (110) about one or more organic substances;
generating a database (122) by storing the information (110) about the one or more organic substances based on a plurality of parameters; and
applying at least one filter (930) to the database (122) to generate output information (130) about a target substance configured to be used in the lithium secondary battery (100), the target substance selected from the one or more organic substances,
wherein the target substance is configured to form at least a part of a low-resistance solid electrolyte interphase (SEI) layer on an anode (20) of the lithium secondary battery (100).

2. The method as claimed in claim 1, wherein the plurality of parameters comprises an ion type, a molecular weight, an electron affinity, an ionization energy, a lithium-ion (Li⁺) interaction energy, a LUMO (lowest unoccupied molecular orbital) energy, a LUMO energy of a lithium-ion complex containing the one or more organic substances (110), or an electron affinity of the lithium-ion complex containing the one or more organic substances (110).

3. The method according to any of the previous claims, wherein the at least one filter (930) comprises a first filter (931), and wherein the first filter (931) filters out a candidate material having an ionization energy greater than a predetermined ionization energy threshold.

4. The method as claimed in claim 3, wherein the predetermined ionization energy threshold is related to an ionization energy of an organic solvent previously determined to be used in the lithium secondary battery (100).

5. The method according to any of the previous claims, wherein the at least one filter (930) comprises a second filter (932), and wherein the second filter (932) filters out a candidate material having a lithium-ion interaction energy greater than a first predetermined lithium-ion interaction energy threshold.

6. The method as claimed in claim 5, wherein the first predetermined lithium-ion interaction energy threshold is related to a lithium-ion interaction energy of an organic solvent previously determined to be used in the lithium secondary battery (100).

7. The method according to any of the previous claims, wherein the at least one filter (930) comprises a third filter (933), and wherein the third filter (933) filters out a candidate material having a lithium-ion interaction energy less than a second predetermined lithium-ion interaction energy threshold.

8. The method as claimed in claim 7, wherein the second predetermined lithium-ion interaction energy threshold is related to an interaction energy between an electrolyte additive previously determined to be used in the lithium secondary battery (100), and lithium ions.

9. The method as according to any of the previous claims, wherein the at least one filter (930) comprises a fourth filter (934), and wherein the fourth filter (934) filters out a candidate material having a LUMO energy of a lithium-ion complex less than a predetermined LUMO energy threshold.

10. The method as claimed in claim 9, wherein the predetermined LUMO energy threshold is related to a LUMO energy of a lithium-ion complex comprising an organic solvent previously determined to be used in the lithium secondary battery (100).

11. The method according to any of the previous claims, wherein the at least one filter (930) comprises a fifth filter (935), wherein the fifth filter (935) filters out a candidate material having an electron affinity of a lithium-ion complex greater than a predetermined electron affinity threshold, and wherein the electron affinity threshold is related to an electron affinity threshold of a lithium-ion complex comprising an organic solvent previously determined to be used in the lithium secondary battery (100).

12. The according to any of the previous claims, further comprising:
receiving an input for selecting at least one of the plurality of parameters; and
receiving information about a numerical range for the at least one of the plurality of parameters,
wherein the at least one filter (930) comprises a sixth filter (936), and
wherein the sixth filter (936) filters out a candidate material in which the numerical range comprises information related to the at least one of the plurality of parameters.

13. The method according to any of the previous claims, wherein the generating of the output information (130) comprises:
receiving an input of selecting at least one of the plurality of parameters on a user interface (1000); and
generating output information (130) about the at least one of the plurality of parameters selected from the output information about the target substance onto the user interface (1000).

14. A computer program stored on a computer-readable recording medium, the computer program comprising instructions which, when the program is executed by a computer, cause the computer to execute the method according to any of the previous claims.

15. An information processing system (230) comprising:
a communication module (336);
a memory (332); and
at least one processor (334) connected to the memory (332) and configured to execute at least one computer-readable program comprised in the memory (332),
wherein the at least one program is preferably configured according to claim 14 and/or causes the information processing system (230) to execute the method according to any of claims 1 to 13, and
wherein the at least one program comprises instructions for:
receiving information (110) about one or more organic substances;
generating a database (122) by storing the information (110) about the one or more organic substances based on a plurality of parameters; and
applying at least one filter (930) to the database (122) to generate output information (130) about a target substance configured to be used in the lithium secondary battery (100), the target substance selected from the one or more organic substances,
wherein the target substance is configured to form at least a part of a low-resistance solid electrolyte interphase (SEI) layer on an anode (20) of the lithium secondary battery (100).
